Europäisches Patentamt

European Patent Office

Office européen des brevets

(11) Publication number: **0 174 204 B1**

(12) **EUROPEAN PATENT SPECIFICATION**

(45) Date of publication of patent specification: **21.11.91**      (51) Int. Cl.⁵: **A61K 39/40**, C12P 21/08, C12N 5/00

(21) Application number: **85306329.5**

(22) Date of filing: **05.09.85**

(54) **Gram-negative bacterial endotoxin blocking monoclonal antibodies and cells producing the same and formulations containing the same, and the production of all thereof.**

(30) Priority: **05.09.84 US 647611**
      **26.04.85 US 727821**

(43) Date of publication of application:
      **12.03.86 Bulletin 86/11**

(45) Publication of the grant of the patent:
      **21.11.91 Bulletin 91/47**

(84) Designated Contracting States:
      **AT BE CH DE FR GB IT LI NL SE**

(56) References cited:
      **EP-A- 0 105 804**
      **WO-A-84/04458**
      **WO-A-85/01659**
      **WO-A-85/02685**

      **CHEMICAL ABSTRACTS, vol. 101, no. 17,
      22nd October 1984, page 532, no. 149444j,
      Columbus, Ohio, US; L.M. MUTHARIA et al.:
      "Monoclonal antibodies specific for Es-
      cherichia coli J5 lipopolysaccharide: cross-
      reaction with other Gram-negative bacterial
      species", & INFECT. IMMUN. 1984, 45(3),
      631-6**

(73) Proprietor: **CETUS CORPORATION**
      **1400 Fifty-Third Street**
      **Emeryville California 94608(US)**

(72) Inventor: **Larrick, James W.**
      **Star Route Box 48**
      **Woodside California 94062(US)**
      Inventor: **Raubitschek, Andrew A.**
      **4301 Jones Bridge Road**
      **Bethesda Maryland 20814(US)**

(74) Representative: **Bizley, Richard Edward et al**
      **HEPWORTH LAWRENCE BRYER & BIZLEY**
      **2nd Floor Gate House South West Gate**
      **Harlow, Essex CM20 1JN(GB)**

EP 0 174 204 B1

CHEMICAL ABSTRACTS, vol. 102, no. 3, 21st January 1985, page 521, no. 22552h, Columbus, Ohio, US; M.J. NELLES et al.: "Mouse monoclonal antibodies reactive with J5 lipopolysaccharide exhibit extensive serological cross-reactivity with a variety of Gram-negative bacteria", & INFECT. IMMUN. 1984, 46(3), 677-81

PROC. NATL. ACAD. SCI. USA, vol. 82, March 1985, pages 1790-1794, US; N.N.H. TENG et al.: "Protection against Gram-negative bacteremia and endotoxemia with human monoclonal IgM antibodies"

2

**Description**

This invention is in the field of biotechnology and relates to somatic cell hybridization and immunology. More particularly, it concerns: monoclonal antibodies that bind to gram-negative bacterial endotoxins and block the biological effects thereof; hybrid cell lines that produce the antibodies; and treatment of gram-negative bacteremia and sepsis with the antibodies.

Bacteremia due to gram-negative bacteria is a major public health problem that results in a substantial number of deaths per year. Symptoms of bacteremia include: fever, leukopenia and hypoglycemia, hypotension and shock, impaired perfusion of essential organs, activation of C3 and the complement cascade, intravascular coagulation and death.

These effects of bacteremia are attributed to the endotoxin (cell-wall lipopolysaccharide (LPS)) of the infecting organism. These LPSs are composed of three regions: Serotype-specific polysaccharide (O-antigen), core polysaccharide, and lipid A. The O-antigen region is made up of repeating oligosaccharide combinations that define type-specific haptenic determinants. The core region is composed of an outer core of hexoses (N-acetylglucosamine, glucose, galactose), and an inner core of heptose, ethanolamine and 2-keto-3-deoxyoctonate (KDO). This region is more conserved among bacterial species than the O-antigen region but does exhibit limited intraspecies and interspecies variation. Lipid A is composed of diglucosamine-4-phosphate, long chain fatty acids, and ethanolamine. KDO forms the link between lipid A and the core region.

Mutants of gram-negative bacteria that lack the O antigen are called "rough" or "R" forms because they do not form smooth colonies on solid media. Several chemotypes of mutant LPSs are known such as Ra, Rb, Rc, Rd, and Re. In chemotype Rc the enzyme UDP-galactose-4-epimerase is deficient so that glucose, but not galactose, is synthesized and incorporated into the core. Re chemotypes lack the O and core regions up to KDO. Ziegler, E. J., et al., N. Engl. J. Med. (1982) 307:1225-1230, describe the preparation of human antisera to the E. coli Rc mutant J5 by vaccinating healthy patients with heat-killed J5. The antisera were used to treat bacteremia and were reported to block the biological effect of LPS.

Kohler, G. and Milstein, C., Nature (1975) 256:495-497, pioneered the use of somatic cell hybridization to make continuous hybridomas that produce monoclonal antibodies. Their work used plasmacytomas and lymphocytes of murine origin. Mutharia et al., Infect. Immun. (1984) 45:631-636, and Nelles et al., Infect. Immun. (1984) 46:677-681, describe LPS cross-reactive murine monoclonal antibodies.

Subsequent investigators have reported applying Kohler and Milstein's techniques to human cells, for example, Croce, C. M., et al., Nature (Lond) (1980) 288:488 and Olsson, L. and Kaplan, H. S., PNAS (USA) - (1980) 77:5429. The literature reports the preparation of human monoclonal antibodies directed against various antigens. EP 44,722 describes a mutant myeloma cell line which, after fusion with antigen-sensitized human spleen B-lymphocytes, yields monoclonal antibody-secreting human-human hybridomas. Also, it suggests making antibodies to "pathogen surface antigens" and "toxins." In addition, Foung et al., J. Immun. Meth. (1984) 70:83-90, discloses human monoclonal antibody production from an EBV-transformed B cell line by fusion to a human-mouse hybridoma. Several fusion partners are described by D. Buck et al., Chapter 11 in Monoclonal Antibodies and Functional Cell Lines, ed. by R. Kennett et al., Plenum Publishing Corp., 1984 and by Larrick and Buck, Biotechniques (1984) 2:6-14. Europ. Pat. Publications 107,528 and 105,804 describe cell lines capable of producing human monoclonal antibodies against a bacterial toxin. In addition, GB 2,086,937; GB 2,113,715; EP 57,107; EP 62,409; EP 118,893; EP 124,301 and EP 131,878 all relate to manufacture of human monoclonal antibodies from hybrid cells.

The antibodies of the invention are characterized in that

(a) their population is substantially homogeneous;

(b) they bind strongly to determinants that are defined by the lipid A of the cell-wall lipopolysaccharides of either E. coli Rc mutants or Salmonella Re mutants;

(c) they bind to either the E. coli Rc mutant lipid A determinants or the Salmonella Re mutant lipid A determinants in intact LPS and in whole gram-negative bacteria;

(d) they block the adverse biological effects of the lipopolysaccharides; and

(e) exhibits the above properties (a) to (d) at substantially the same extent as the antibody produceable by any of the hybridomas obtainable from ATCC under deposition numbers HB 8598, HB 8607, HB 8735, HB 8733, HB 8736 or HB 8734.

Stable, permanent hybrid cell lines that produce the abovedescribed antibodies and progeny of those lines, and a specific mouse x human B-lymphocyte fusion partner producing such cell lines and partially adapted to serum-free medium are another aspect of the invention.

The invention also contemplates formulations for treating bacteremia or sepsis comprising a therapeutically effective amount of one or more of the above-described antibodies. Preferred compositions comprise two or more of the antibodies each of which binds to a different determinant located as specified in (b) above.

These and other aspects of the invention are described in detail below.

Figure 1 shows the growth curve of D-234 cells in serum-free media HL-1 (Ventrex Labs, Portland, Me) spinner culture. The circles represent IgM levels, the triangles represent cell yield, and the squares represent glucose levels.

Figure 2 shows the growth curve of D-267 cells in serum-free media HB104 (Hana Biologicals, Berkeley, CA) spinner culture, where the circles, triangles and squares are as in Figure 1.

As used herein the term "cell line" refers to individual cells, harvested cells, and cultures containing cells so long as they are derived from cells of the cell line referred to.

As used herein with respect to hybrid cell lines the term "progeny" is intended to include all derivatives, issue, and offspring of the cell line regardless of generation or karyotypic identity. As used herein the term "monoclonal antibody" refers to an antibody selected from antibodies whose population is substantially homogeneous, i.e., the individuals of the antibody population are identical except for naturally occurring mutations.

As used herein with respect to a given monoclonal antibody the term "functional equivalent" means a monoclonal antibody that recognizes the same determinant as and crossblocks the monoclonal antibody referred to. It is intended to include antibodies of the same or different immunoglobulin class and antigen binding fragments (e.g., Fab, F(ab')$_2$, Fv) of the monoclonal antibody.

As used herein with respect to administering antibody to patients the term "treat" and conjugates thereof refers to therapy and/or prophylaxis.

As used herein with respect to characterizing the claimed hybrid cell lines the terms "permanent" and "stable" mean that the lines remain viable over a prolonged time, typically at least about six months, and maintain the ability to produce the specified monoclonal antibody through at least about 25 passages.

As used herein with respect to characterizing the monoclonal antibodies herein the term "binds strongly" means that the antibody exhibits a relatively strong binding affinity to lipid A determinants of the E. coli Rc mutant LPS or Salmonella Re mutant LPS as compared to the affinity of an antibody as described by Teng et al., Proc. Natl Acad Sci USA, Vol. 82, PP 1790 - 1794 (March 1985) for either of these two determinants, as measured by bacterial ELISA.

As used herein with respect to describing LPS, the term "intact" means that the LPS has 'O' antigen carbohydrates.

As used herein the term "whole gram-negative bacteria" means any gram-negative bacteria with all of its component parts, not just the intact or core LPS, lipid A or rough mutant portions thereof.

Monoclonal antibodies that meet the functional criteria of the invention (specific bacteria binding, endotoxin blocking) may be made using cells of diverse mammalian origin. Rat and human embodiments have been made. The antibodies may be of any isotype, including IgG and IgM, with IgM types being specifically exemplified herein. The human embodiments are the products of triomas synthesized by somatic cell hybridization using a mouse x human parent hybrid cell line and Epstein-Barr virus (EBV)-transformed human PBLs or splenocytes from non-immunized volunteers or volunteers immunized with available gram-negative bacterial vaccines or inactivated gram-negative bacteria. Fresh PBLs or splenocytes (not transformed) may be used, if desired. The rat embodiments are the products of hybridomas synthesized by somatic cell hybridization using a rat myeloma line and splenocytes from rats immunized with an E. coli Rc mutant.

A preferred strategy for preparing and identifying hybrids that produce antibodies of the invention follows. Cells (PBLs, splenocytes, etc.) are panned an LPS coated tissue culture plates, then EBV transformed and fused to the tumor fusion partner (mouse myeloma x human B cell or rat myeloma). Panning involves incubation of the population of immunocompetent cells on a plastic surface coated with the relevant antigen. Antigen-specific cells adhere. Following removal of non-adherent cells, a population of cells specifically enriched for the antigen used is obtained. These cells are transformed by EBV and cultured at 10$^3$ cells per microtiter well using an irradiated lymphoblastoid feeder cell layer. Supernatants from the resulting lymphoblastoid cells are screened by ELISA against an E. coli Rc LPS and a Salmonella Re LPS. Cells that are positive for either Rc or Re Lipid A LPS are expanded and fused to a 6-thioguanine-resistant mouse x human B cell fusion partner. If the mouse x human B cell fusion partner is used, hybrids are selected in ouabain and azaserine. Supernatants from the Rc or Re positive hybrids are assayed by ELISA against a spectrum of gram-negative bacteria and purified gram-negative bacterial LPSs. Cultures exhibiting a wide range of activity are chosen for in vivo LPS neutralizing activity. Many but not all antibodies so produced are of the IgM class and most demonstrate binding to a wide range of purified lipid A's or rough LPS's. The antibodies demonstrate binding to various smooth LPS's and to a range of clinical bacterial isolates by ELISA.

As used herein the term "neutralizing" is used to denote the ability of an antibody to block the adverse biological effects of gram-negative bacteria endotoxin in vitro or in mammals regardless of the particular mechanism involved. It is intended to include, without limitation, mechanisms in which the antibody affects the biological activity of the endotoxin by binding thereto, causes the endotoxin to be degraded, or affects the activity of the endotoxin by altering the kinetics and/or site of its clearance. Neutralizing activity may be assayed in vivo using a murine model. Balb/C mice caged at 37° C, for example, may be injected ip or iv with a lethal dose of LPS (LD$_{50}$) at which 50% of the mice die (approximately 1-10 μg). Antibody may be injected ip or iv at 0.2 to 20 mg/kg before or after the LPS injection. The neutralizing effect is determined by comparing the morbidity of test mice with that of control mice (e.g., mice given no antibody, mice given non-binding antibody, etc.).

The hybridomas that produce the invention antibodies may be grown in suitable culture media such as Iscove's media or RPMI-1640 medium (Gibco, Grand Island, NY) or in vivo in immunodeficient laboratory animals. If desired, the antibody may be separated from the culture medium or body fluid, as the case may be, by conventional techniques such as ammonium sulfate precipitation, ion exchange chromatography, affinity chromatography, electrophoresis, microfiltration, and ultracentrifugation.

The monoclonal antibodies of this invention may be used passively to immunize individuals who suffer from bacteremia or sepsis or who are at risk with respect to bacterial infection. Preferably a plurality of different monoclonal antibodies, each of which recognizes and binds to a distinct determinant of the cell wall LPS located interiorly of the core region are employed. In such treatment the antibody/antibodies will normally be administered parenterally (e.g., itravenously, intraarterially, intramuscularly, intraperitoneally), preferably intravenously. The dose and dosage regimen will depend upon whether the antibody/antibodies is/are being administered for therapeutic or prophylactic purposes, the patient, and the patient's history. The total amount of an antibody adminstered per dose will typically be in the range of about 0.1 to 20 mg/kg of patient body weight, preferably 0.1 to 10 mg/kg of patient body weight.

For parenteral administration the antibody/antibodies will be formulated in a unit dosage injectable form (solution, suspension, emulsion) in association with a pharmaceutically acceptable parenteral vehicle. Such vehicles are inherently nontoxic and nontherapeutic. Examples of such vehicles are water, saline, Ringer's solution, dextrose solution, and 5% human serum albumin. Nonaqueous vehicles such as fixed oils and ethyl oleate may also be used. Liposomes may be used as carriers. The vehicle may contain minor amounts of additives such as substances that maintain isotonicity and chemical stability, e.g., buffers and preservatives. The antibody will typically be formulated in such vehicles at a concentration of about 1.0 mg/ml to 100 mg/ml.

The various aspects of the invention are further described by the following example. This example is not intended to limit the invention in any manner. In the example, all temperatures are in degrees Celsius.

Fusion Partners

All cell lines were maintained in Iscove's DME medium supplemented with 10% fetal bovine serum (FBS), 2mM glutamine and 5 x 10$^{-5}$ M 2-mercaptoethanol. The cell lines were checked routinely for the presence of mycoplasma. For large-scale production of human monoclonal antibodies, cell lines were adapted to serum-free growth in HL-1 medium obtained from Ventrex Labs, Portland, ME, and in HB104 medium obtained from Hana Biologicals, Berkeley, CA.

A. Human B Lymphocytes

Volunteers with naturally acquired high titer serum antibodies to E. coli J5 or S. minnesota R595 core glycolipids or vaccinated with a standard available typhoid injection to produce high LPS antibody titers were used as sources of peripheral blood lymphocytes. Fifty ml of venous blood was drawn from the volunteers on days 5 and 7. The blood was centrifuged, the buffy coat was harvested, and the harvested buffy coat was gradient centrifuged using Ficoll/Hypaque to separate lymphocytes. Ficoll/Hypaque in a 1 liter quantity is prepared by dissolving 64 g of Ficoll (Sigma) in 600 ml of distilled water using a stir bar rotor at low speed and then adding 99 g of diatrizoate sodium (Sterling Drug, N.Y.). After both substances were dissolved, more water was added to 1 liter volume and 0.7 g NaCl was added. T cells were removed by aminoethylthiouronium treated sheep red blood cells (AET-SRBC) rosetting, using the technique described by Madsen and Johnsen, J. Immunol. Meth. (1979) 27:61-74. The remaining B cell-enriched lymphocyte population was transformed with Epstein-Barr virus using the technique described by Foung et al., J. Immunol. Meth. (1984) 70:83-90, except that cells were generally cultured at 10$^{3}$-10$^{4}$ cells/well in 96-well culture plates.

The cultures were assayed after 20 days by ELISA using E. coli J5 as antigen (described below). The cells in one well exhibiting a high antibody titer were subcultured at 1000 cells/well, then at 500 cells/well, and positive cultures containing core-specific, antibody-secreting cells were pooled.

Alternatively in a preferred embodiment, the mononuclear cells were isolated from the peripheral blood and were cultured on plastic to deplete plastic-adherent monocytes. The remaining cells (T and B) were panned on plates coated with LPS of S. minnesota Re R595 obtained from Ribi ImmunoChemResearch, Inc. Adherent cells were then transformed by EBV and maintained in culture or cultured on microtiter plates and screened for those positive for Re LPS. Those cells maintained in culture were panned again ten days later on LPS of E. coli J5 from Ribi. Adherent cells were collected and sorted by cell sorter for gamma-positive cells. These $\gamma$-selected cells were grown up and tested positive for IgG production but negative for Re LPS reactivity. To select potential Re-positive cells this population was sorted into microtiter plates by cell sorter.

Panning on a specific antigen can at least double the number of recoverable antigen-specific cells and can improve the secretion rate of antibody.

## B. Rat Splenocytes

E. coli Rc mutant J5 in saline at $10^9$ cells/ml was used as antigen. Rats were injected ip and sq with 0.5 ml of bacterial suspension + 0.5 ml of complete Freund's adjuvant (CFA). The rats were boosted on day 25 with an identical injection and on day 29 with 0.2 ml of bacterial suspension injection iv. Splenectomies were carried out on day 32.

## C. F3B6 (Mouse x Human Line)

A mouse-human heterohybrid fusion partner designated F3B6 (adapted to 99% serum-free medium and deposited with the ATCC under ATCC Accession No. HB8785 on April 18, 1985) was constructed by fusing human peripheral blood lymphocyte (PBL) B cells obtained from a blood bank with the murine plasmacytoma cell line NS1 obtained from ATCC under ATCC No. TIB18(P3/NS1/1-AG4-1). The PBL cells from random buffy coat were transferred to a 50 ml centrifuge tube and diluted with 30 ml Hanks' balanced salt solution ($Ca^{2+}$-free/$Mg^{2+}$-free) (HBSS-/-). Then 10 ml Ficoll-Hypaque was added and the mixture centrifuged at 1500 rpm for 15 minutes at room temperature. The interface was removed and the mixture was washed with HBSS-/- and resuspended in HBSS-/-. The cells were counted.

The NS-1 cells were grown in 4 x 175 flasks and harvested, washed with HBSS-/- and resuspended in HBSS-/-. The cells were counted.

Approximately $5 \times 10^7$ B-cells and $2.5 \times 10^7$ NS-1 cells (2:1 ratio) were added to each of 5-50 ml centrifuge tubes for fusion. The mixture was centrifuged at 1200 rpm for eight minutes at room temperature to form a tight pellet. All of the supernatant was removed and the tube was kept at 37°C for further manipulations. A total of 1 ml of warm 50% polyethyleneglycol of molecular weight 1540 (PEG 1540) (BDH Chemicals, Poole, England) was added over a one minute period using a 1 ml pipette. The cell pellet was gently stirred with the tip of the pipette as the PEG was being added. Then 1 ml of HBS-/-was added at 37°C over a one-minute period to dilute gradually the PEG. The cells were washed twice with HBSS-/- and resuspended in Iscove's medium in several T150 flasks.

On day 2 the cells were washed in HBSS-/- in 50 ml centrifuge tubes. A total of 10 ml of Ficoll-Hypaque was added to the tubes. The tubes were centrifuged at 1500 rpm for 15 minutes at room temperature and the live cells at the interface were removed. The pellet was washed twice with RPMI-1640 (Gibco) and resuspended in an enriched hypoxanthine/azaserine selection medium (EHA) consisting of 100 μM hypoxanthine (Sigma), 5 μg/ml azaserine (Sigma) and Iscove's medium (Gibco), 10% NCTC (M. A. Biologicals), 20% heat-inactivated-FBS. The density was adjusted to $2.5 \times 10^4$ cells/ml medium.

At day 5 the suspensions were washed twice with HBSS-/- and resuspended in 10 ml Iscove's medium. Live cells were separated by Ficoll-Hypaque density gradient centrifugation as described above. Cells were washed twice with RPMI-1640 + 20% FBS, and then plated out in 96-well plates at $10^5$ cells/ml. At days 7, 9 and 12 the EHA selection medium described above was added each time. At days 15 and 18 the plates were fed with EHMT medium containing hypoxanthine, methotrexate and thymidine. The supernatants were assayed for Ig secretion and Ig secreting hybrid cell lines were cloned by limiting dilution in U bottom 96 well plates.

Well F3B6 was selected for 6-thioguanine selection. Several roller bottles of F3B6 were grown up. A total of 10 μg/ml of 6-thioguanine was added to the roller bottles. Dead cells were removed by Ficoll-Hypaque density gradient centrifugation on days 2, 5 and 7. A 6-thioguanine resistant clone was grown up.

Test fusions were performed, and the cell line was tested for ouabain resistance.

The resultant cell line was adapted to growth and maintenance in 99% serum-free medium and 1% FBS for more reproducible manufacturing by the following multi-step process:

1. Two days prior to subculturing, the cells were fed with 50% of the Iscove's DME in which they were growing, (conditioned medium), and 50% by weight of serum-free medium HL -1 prepared in-house from powder supplied by Ventrex, Inc.

2. Two days later, or when the hybridoma cells reached densities of $8 \times 10^5$ to $1 \times 10^6$ cells/ml, the cells were subcultured and planted with 50% of Iscove's DME medium and 50% of the serum-free medium. The cells were removed from the latter medium by centrifugation at 200 x g for five minutes. The Iscove's DME medium was mixed with 50% of the serum-free medium to form a 50:50 mixture, in which the cell pellet was suspended and then counted. An appropriate amount of cell suspension was planted in the vessel with 50% Iscove's DME and 50% serum-free medium. The planted cell densities preferably do not fall below $5 \times 10^4$ cells/ml and not exceed $1 \times 10^5$ cells/ml.

3. After two to three days post-planting, or when the cell density reached $8 \times 10^5$ to $1 \times 10^6$ cells/ml, the cells were refed with 50% Iscove's DME and 50% serum-free medium.

4. Step 3 has repeated for another passage.

5. After two to three days in culture or when the cell density reached $8 \times 10^5$ to $1 \times 10^6$ cells/ml and viability was about 85%, the cells were cultured on serum-free medium only. When the cells were planted in the serum-free medium for the first time the cell densities were between $1 \times 10^5$ to $8-9 \times 10^5$ cells/ml. The final medium HL-1 with 1% FBS.

## D. Rat Tumor Line

An available rat tumor line was used.

## Fusion Protocol

The fusion mixture contained polyethylene glycol (PEG) 4000, 40% (w/v); dimethylsulfoxide (DMSO), 10% (v/v) and 5 $\mu$g/ml poly-1-arginine in Hanks' balanced salt solution (HBSS)-/+ ($Ca^{2+}$-free, 2mM NgSO$_4$). Forty g of PEG 4000 was combined with 10 ml of DMSO and 50 ml of HBSS-/+. The mix was autoclaved for 25 minutes. Before use, the pH of the fusion mixture was adjusted to between 7.5 and 8.5 with sterile 0.1 N NaOH.

Plates (6-well cluster, 35 mm well diameter) were prepared as follows: 2 ml of HBSS-/+ and 50 $\mu$l of a filter sterilized, 20-100 $\mu$g/ml, peanut agglutinin (PNA, Sigma) were added to each well. Plates were incubated at 37° C for at least one hour prior to use. PNA stock was stored frozen, and a freshly thawed aliquot was used to coat fusion cells. Smaller sized wells were used if cell numbers were limited.

Parent cells in Ficoll-Hypaque were washed twice in HBSS-/+ at room temperature and subsequently resuspended and combined at a ratio of 5:1 to 1:1 lymphocyte-splenocyte:fusion partner in HBSS-/+ warmed to 37° C. Two ml of the combined cell suspension ($3 \times 10^7$ cells/well) was added to each pretreated well containing 1 $\mu$g/ml PNA coating solution. The wells were incubated at 37° C for one minute. Plates were spun onto bottom of the plate at 400-500 x g, room temperature, for five minutes to form a monolayer of cells. Supernatant was then aspirated off the plates, leaving behind adherent coating of cells.

Two ml of PEG fusion mixture described above and warmed to 37° C was carefully added down the side of the fusion cell. After one minute, the PEG solution was diluted with a fusion dilution mixture (FDM) of 5% DMSO (Sigma) HBSS-/+ (warmed to 37° C and filter sterilized) at a rate of 2 ml/min (0.5 ml every 15 sec) for the next two-three min (4-6 ml). For the next two minutes the FDM was added at a rate of 4 ml/min with mixing. FDM was always added down the side of the well, so as not to disturb the monolayer, and the plates were swirled constantly to ensure optimal mixing.

At the end of the two minutes the wells were aspirated to remove diluted PEG fusion mixture. The remaining film of PEG mixture was diluted at a rate of 2 ml/min for one-two min with warm FDM. Again the plate was constantly swirled. Over a period of 0.25-2 minutes with swirling, 5 ml of HBSS-/+ warmed to 37° C was added to the fusion well at a rate of 1 ml/15 sec. The well was then filled up with HBSS-/+ and all supernatant was aspirated from the monolayer. Finally, each fusion well was washed once or twice with about 5-10 ml of warm HBSS-/+, aspirated and washed again with about 5 ml of HBSS-/+ and aspirated. Five ml of warm Iscove's complete medium and 15-20% FBS, were added to each well, and the plates were incubated at 37° C for 24 hours. The day following fusion the cells were resuspended at a density of $10^5$ cells/ml in EHA medium containing azaserine (2 $\mu$g/ml), hypoxanthine (100 $\mu$M), and ouabain (1 $\mu$M) and plated at 0.1 ml/well in 96-well plates. Cultures were subsequently fed every three days. Growing hybrids were visible by day 10.

ELISAs

A. Bacteria

Fifty-100 $\mu$l of 0.5-1.0% glutaraldehyde (Sigma) in deionized water was coated onto flat/bottom microtiter plates (Dynatech). After one to four hours of incubation at room temperature the wells were aspirated or washed twice with distilled water. The bacteria were washed in saline and reconstituted to 0.25% (v/v). Sixty-100 $\mu$l of this bacterial suspension was added per well and the plates were spun for 20 minutes at 2000 rpm. The suspensions were incubated overnight or for a minimum of two hours. The plates were then washed with 100 $\mu$l phosphate buffered saline containing 0.1 g/liter $MgSO_4$ and 0.1 g/liter $CaCl_2$ - ($PBS^{++}$), 0.05% Tween 20 surfactant (Sigma), and preferably 0.01% thimerosal and 1% bovine serum albumin (BSA) (Sigma). The supernatant was incubated for 90 minutes at room temperature and washed three times with $PBS^{++}$, Tween 20 and thimerosal. Then the plate bottom was optionally blotted with soft tissue. Fifty-100 $\mu$l of horseradish peroxidase-conjugated goat anti-human IgG (Tago, Inc.) or horseradish peroxidase-conjugated goat anti-human IgM (Jackson Labs) was then added to each well. The wells were incubated at room temperature or $40°C$ for 30 minutes and washed up to five times with $PBS^{++}$, 0.05% Tween 20 and 0.01% thimerosal, and optionally blotted. Two-hundred $\mu$l of ABTS substrate was then added to each well, the substrate consisting of 55 mg/ml of ABTS aqueous stock solution diluted 1:1000 with 0.1 M sodium citrate buffer at pH 4.5 to which 1:1000 of 30% $H_2O_2$ was added immediately before use. Each well was incubated for 30 minutes at $37°C$ in the dark. The contents of the wells were transferred to a transparent plate and were read with an ELISA reader at 405 nm. Readings were reported on a scale of 1 to 10 with 1 = 0.2 OD, 10 = 2.0 OD.

B. LPS

Flat-bottom microtiter plates were coated overnight with 50 $\mu$l of a preparation of sonicated LPS, 50 $\mu$g/ml in 0.05 mM sodium bicarbonate buffer, pH 9.6. Plates were washed with $PBS^{++}$ with 0.05% Tween 20 (Sigma), and preferably 0.01% thimerosal up to five times by immersion or with an automated plate washer. Subsequently, 100 $\mu$l of of $PBS^{++}$, 1% BSA, 0.05% Tween 20, and preferably 0.01% thimerosal was added to each well, followed by 100 $\mu$l of the test supernatants. Supernatants were incubated for 30 minutes at $4°C$ to room temperature and then washed up to five times with the $PBS^{++}$/Tween/thimerosal mixture. Then a total of 50-100 $\mu$l of peroxidase-conjugated goat anti-human IgM (Tago) diluted in $PBS^{++}$, BSA, Tween 20 and thimerosal was added and the mixture incubated for 30 minutes at room temperature or $40°C$ and washed up to five times. Then 200 $\mu$l of the ABTS peroxidase substrate described for the bacterial ELISA was added to each well and each well was incubated for 30 minutes at $37°C$ in the dark. The contents of the wells were read on a plate ELISA reader at 405 nm.

C. IgM

Immulon II flat-bottom microtiter plates were coated at 100 $\mu$l/well with goat anti-human IgM (Tago) diluted 1:100 in 50 mM bicarbonate buffer (pH 9.6). After 90 minutes at $37°C$, plates were washed with $PBS^{++}$, 0.05% Tween 20, and preferably 0.01% thimerosal up to five times by immersion or with automated plate washer. Then 100 $\mu$l of $PBS^{++}$, 1% BSA, 0.05% Tween 20, 0.01% thimerosal was preferably added to each well. A total of 100 $\mu$l of test supernatant was added to first wells and preferably duplicate two-fold dilutions were made. One well was preferably left as control. The plates were incubated for 30 minutes at $22°C$ and then washed up to five times as described above. Then, a total of 50-100 $\mu$l of peroxidase-conjugated goat anti-human IgM antibody (Tago) diluted in $PBS^{++}$, BSA, Tween 20 and thimerosal was added and the mixture incubated for 30 minutes at room temperature or $40°C$ and washed up to five times. Then a total of 200 $\mu$l of the ABTS peroxidase substrate described for the bacterial ELISA was added to each well. The mixture was incubated for 30 minutes at $37°C$ in the dark and read on an ELISA plate reader ($OD_{405}$) using as IgM standard pooled human myeloma (Cappell) previously standardized versus a Tago Standard.

Hybrid Screening

A. B Lymphocyte x F3B6

8

Culture supernatants were assayed by ELISA as described above using commercial E. coli J5 and S. minnesota Re595 LPS's (obtained from List Biologicals or Ribi ImmunoChemResearch, Inc.). Positive wells were subcloned in soft agar or by limiting dilution and reassayed approximately two weeks later. Limiting dilution cloning was performed in 96-well U-bottom plates in Iscove's DME medium with 20% FBS. For soft agar cloning 1000 cells in 1 ml of 0.33% agarose (FNC Corp.), made in Iscove's DME medium/20% fetal calf serum, were placed over a bed of 4 ml of agarose (0.4%) in 60 mm culture dishes.

Selection for a nonproducer parent cell line or a high-producer hybrid was accomplished using a reverse-plaque technique. Protein A-coated sheep erythrocytes (1.0%) were added to the upper layer of soft agar according to the method of Gronowicz et al., Eur. J. Immunol. (1976) 6:588-590.

Sixteen hybrids were chosen based on their titers of anti-Re and anti-J5 antibody for expansion and further testing.

Sixteen human monoclonal antibodies produced from these hybrids were isotyped using the IgM ELISA mentioned above and all were of the IgM class. All hybrids have been cloned and stably produce greater than 10 µg of antibody per ml of spent culture media. Table I presents the ELISA results of these anti-LPS hybridomas binding to various purified core lipopolysaccharides commercially obtainable from Ribi ImmunoChemResearch, Inc. D253 is a negative control human monoclonal antibody which does not bind LPS but binds P. aeruginosa toxin A. Table II demonstrates binding of these monoclonal antibodies to various rough mutant bacteria commercially obtainable from Ribi ImmunoChemResearch, Inc. Table III demonstrates binding of these same antibodies to a variety of bacteremic gram-negative clinical isolates. This series of sixteen antibodies shows a number of different binding patterns:

1. Some of the antibodies recognize only core antigenic determinants;

2. Some recognize core antigenic determinants as well as determinants found on certain smooth lipopolysaccharide molecules; and

3. Some antibodies demonstrate broad cross-reactivity to not only core antigenic determinants, but to O-antigenic determinants found on whole bacteria.

## TABLE I

### CORE LIPOPOLYSACCHARIDES

| Anti-body | E. coli J5 (Rc) | Salmonella minn. R595 (Re) | S. typhi Re | E. coli J5 (Rc) Lipid A | S. minn. R595 (Re) Lipid A | S. typhi Lipid A |
|---|---|---|---|---|---|---|
| D253* | 0** | 1 | 1 | 0 | 1 | 1 |
| D234 | 7 | 7 | 4 | 7 | 1 | 9 |
| D267 | 3 | 6 | 1 | 3 | 1 | 7 |
| D250 | + | | | | | |
| D244 | + | + | | | | |
| | | | | | | |
| L116 | 8 | 9 | 0 | 8 | 9 | 8 |
| L118 | 8 | + | 2 | 9 | 9 | + |
| L119 | 9 | + | 6 | + | 9 | + |
| L121 | 9 | 8 | 9 | 9 | 8 | + |
| L123 | 8 | 9 | 8 | 9 | 9 | 9 |
| L124 | 0 | 3 | 0 | 1 | 3 | 1 |
| L126 | 0 | 7 | 0 | 3 | 8 | 0 |
| | | | | | | |
| WI-3 | 8 | + | 3 | + | 9 | 9 |
| WI-4 | 3 | + | 0 | + | 9 | + |
| WI-5 | 4 | 9 | 1 | 8 | 8 | 7 |
| WI-6 | 6 | + | 0 | 9 | + | + |
| WI-7 | 7 | 8 | 0 | + | + | + |

\* Negative control
\*\* Magnitude of numbers indicates the degree of monoclonal binding.
0 is negative, + is off scale with the plate reader set at 2.0
at 2.0 absorbance full scale.

EP 0 174 204 B1

## TABLE II

### ROUGH MUTANT - BACTERIA

| Antibody | SL3770† | SL3749 | SL3750 | SL3748 | E. coli J5 (Rc) | SL3789 | S. minn. R595 (Re) |
|---|---|---|---|---|---|---|---|
| D253* | 0** | 0 | 0 | 0 | 0 | 0 | 0 |
| D234 | 7 | + | + | + | + | + | + |
| D267 | 1 | 4 | 5 | 7 | 6 | 3 | 7 |
| D250 | ND | ND | ND | ND | 9 | ND | 0 |
| D244 | 0 | 1 | 0 | 3 | 5 | 1 | 8 |
| L116 | 0 | 0 | 0 | 5 | 0 | 0 | 7 |
| L118 | 0 | 3 | 5 | 9 | 4 | 4 | + |
| L119 | 0 | 0 | 0 | + | 2 | 0 | + |
| L121 | 0 | 0 | 1 | + | + | 0 | + |
| L123 | 0 | 1 | + | + | 9 | 1 | + |
| L124 | 0 | 0 | 0 | 1 | 0 | 0 | 1 |
| L126 | 0 | 0 | 0 | 2 | 0 | 1 | 5 |
| WI-3 | 2 | 5 | 7 | 8 | 8 | 4 | 1 |
| WI-4 | 1 | 2 | 2 | 3 | 2 | 1 | 1 |
| WI-5 | 1 | 1 | 2 | 3 | 2 | 1 | 1 |
| WI-6 | 1 | 1 | 2 | 2 | 2 | 1 | 1 |

† See Lyman et al. (1976) Infect. Immun. 13:1539-1542; SL3770 is smooth; SL3749 and SL3750 are superficial rough (Ra, Rb); SL3748 is Rc and SL3789 is Rc.
\* Negative control
\*\* See legend Table I
ND = not determined

11

## TABLE III

### CLINICAL ISOLATES

| Anti-body | K. pneumoniae SM-1 | P. aeruginosa Type I | P. aeruginosa Type III | E. coli SM-1 | E. coli SM-2 | E. coli SM-5 | E. aerogenes |
|---|---|---|---|---|---|---|---|
| D253* | 0** | ND | 0 | 0 | ND | 0 | 0 |
| D234 | 2 | 1 | 0 | 2 | 0 | 3 | 3 |
| D267 | 0 | 0 | 1 | 0 | 0 | 0 | 0 |
| D250 | 0 | 0 | 0 | 4 | 0 | 1 | 0 |
| D244 | 0 | 0 | ND | 0 | 0 | 0 | 0 |
| L116 | 0 | 0 | 0 | 0 | 0 | 0 | 0 |
| L118 | 7 | 0 | 0 | 8 | 3 | 8 | 8 |
| L119 | 0 | 0 | 0 | 0 | 0 | 0 | 0 |
| L121 | 2 | 0 | 0 | 5 | 0 | 1 | 5 |
| L123 | 1 | 0 | 0 | 3 | 0 | 2 | 3 |
| L124 | 0 | 0 | 0 | 0 | 0 | 0 | 0 |
| L126 | 2 | 0 | 0 | 0 | 0 | 0 | 0 |
| WI-3 | 7 | 7 | 1 | 3 | 4 | 5 | 6 |
| WI-4 | 4 | 7 | 1 | 4 | 3 | 4 | 5 |
| WI-5 | 3 | 5 | 0 | 3 | 1 | 2 | 4 |
| WI-6 | + | 9 | 1 | 9 | 7 | 8 | 9 |
| WI-7 | 4 | 5 | ND | 3 | 1 | 3 | 3 |

\* Negative control
\*\* See legend Table I

Antibody D250 binds only to J5 E. coli (Rc) core determinants with minimal binding to other core lipopolysaccharides and rough mutant bacteria. It gives spotty binding to clinical isolates of E. coli. Antibody D244 binds to E. coli J5 and Salmonella minnesota R595 LPS and bacteria, but not to any other bacteria or lipopolysaccharides. Antibodies D234 and D267 show considerable binding to rough lipopolysaccharides and lipid A's with spotty binding on clinical isolates. The 'L' series and 'W' series of antibodies show high binding on rough lipopolysaccharides with a few 'holes' in the binding patterns. Antibodies within the 'L' series bind to more clinical isolates with a higher binding affinity. In general, those monoclonals showing the highest amount of core LPS or rough bacterial binding demonstrate the most cross-reactivity on clinical isolates.

## TABLE IV

### CORE LIPOPOLYSACCHARIDES

| Antibody Clone No. | E.coli Rc LPS | S. minn. Re LPS | S. typhi Re LPS | E. coli Rc Lipid A | S. minn. Re Lipid A | S. typhi Parent Lipid A | E. coli 0111:B4 LPS | Plastic |
|---|---|---|---|---|---|---|---|---|
| D253-15-6* | 0** | 1 | 1 | 0 | 1 | 1 | 0 | 0 |
| D234-4-27 | 7 | 7 | 4 | 7 | 1 | 9+ | 1 | 0 |
| D267-22-37 | 3 | 6 | 1 | 3 | 1 | 7 | 1 | 0 |
| D250-12-4-3 | 7 | 1 | 7 | 3 | 0 | 8 | 0 | 0 |
| L116-2-4 | 2 | 7 | 0 | 0 | 0 | 7 | 0 | 0 |
| L118-8-4 | 0 | 9+ | 0 | 9 | 2 | 5 | 0 | 0 |
| L118-22-2 | 2 | 9+ | 0 | 9+ | 2 | 9+ | 0 | 0 |
| L119-4 | 8 | 8 | 2 | 2 | 1 | 8 | 0 | 0 |
| L120-1 | 3 | 6 | 1 | 3 | 9 | 5 | 9+ | 2 |
| L121-7 | 9+ | 9+ | 9+ | 9+ | 2 | 9+ | 0 | 0 |
| L123-15 | 7 | 8 | 6 | 8 | 1 | 9 | 0 | 0 |
| L124-3 | 1 | 9+ | 0 | 9+ | 1 | 9+ | 0 | 0 |
| L126-1 | 0 | 7 | 0 | 6 | 1 | 3 | 0 | 0 |
| S261*** | 0 | 1 | 1 | 0 | 0 | 1 | 0 | 0 |

\* Negative control

\*\* Magnitude of numbers indicates the degree of monoclonal binding. 0 is negative and 9+ is the strongest binding with the plate reader set at 2.0 at 2.0 absorbance full scale.

\*\*\* Antibody of Teng et al., Proc. Natl Acad Sci USA, Vol. 82, PP 1790 - 1794 (March 1985).

In another test certain clones of the "L" and "D" series of antibodies, the D253 clone control and an antibody S261 representative of the work of Teng et al., Proc. Natl Acad Sci USA, Vol. 82, PP 1790 - 1794 (March 1985) were evaluated for their binding to purified ore LPS and whole bacteria using the bacteria ELISA test described above. The results of the evaluations using purified core LPS and whole bacteria are provided in Tables IV and V for purified LPS and VI for whole bacteria.

In another experiment, antibody D234 and comparative antibody S261 were tested against various purified core LPS. The results are provided in Table V.

## TABLE V

### CORE LIPOPOLYSACCHARIDES

| Antibody | E. coli Rc LPS | S. minn. Re LPS | E. coli Rc Lipid A | S. minn. Re Lipid A |
|---|---|---|---|---|
| D234 | 7 | -* | - | - |
| S261 | 1 | 1 | 1 | 2 |

\* Magnitude of numbers indicates the degree of monoclonal binding, where the - indicates the strongest binding, which exceeds 9 on the scale.

The antibodies D234 and S261 were tested against the whole bacteria E. coli J5 and S. minnesota Re 595 using the bacterial ELISA test described above. The results are indicated in Table VI.

## TABLE VI

### WHOLE BACTERIA

| Antibody | E. coli Rc LPS | S. minn. Re LPS |
|---|---|---|
| D234 | -* | - |
| S261 | - | - |

\* See legend of Table V

The results in Tables I-VI indicate that all of the monoclonal antibodies produced in accordance with this invention, except L116-2-4, which is not part of the invention in view of its properties, meet the binding criteria defined herein, i.e., they bind strongly to the purified lipid A determinants of E. coli Rc and/or S. minn. Re LPS (Tables I, IV and V) and they bind to these determinants in intact LPS (Table III) and in whole bacteria (Table VI). In contrast, Tables IV and V show that the S261 comparative antibody does not bind or binds very weakly (one-seventh the strength of D234) to the purified lipid A determinants of E. coli Rc and/or S. minn. Re.

In a neutralization experiment, mice were injected intraveneously with a dose of 0.2 ml each of a 1:1 dilution of a hybridoma culture supernatant of negative control antibody D-253 or invention antibody D250, described above. Four hours later the mice were challenged ip with 7, 10-fold dilutions of E. coli J5 core LPS bacteria, five mice per dilution, where before infection the bacteria were suspended in 0.5 ml of hog gastric mucin, 5% in normal saline, to which 15 mg of D-galactosamine had been added. Table VII indicates the results, where $LD_{50}$ is the lethal dose of LPS bacteria at which 50% of the mice die.

## TABLE VII

| Monoclonal Antibody | $LD_{50}$ | Fold Increase in $LD_{50}$ compared to control |
|---|---|---|
| D253 (control) | $9.8 \times 10^4$ | -- |
| D250 | $2.5 \times 10^6$ | 26 |

The results show that the D250 antibody of this invention exhibits statistically significantly more protective activity in mice against the endotoxin core determinants of E. coli J5 than the negative control antibody D253, indicating the neutralizing or blocking property of an invention antibody.

Two of the hybridomas (D234 and D267) were adapted to growth and maintenance in serum-free medium for large-scale, more reproducible spinner culture production of monoclonal antibodies using the following step-wise method:

1. Two days prior to subculturing, the cells were fed with a mixture of the Iscove's DME in which they were growing, 50% of the amount of FBS in the medium in which they were growing, and 50% by weight of serum-free medium HL-1 supplied by Ventrex, Inc. or HB104 supplied by Hana Biologicals.

2. Two days later, or when the hybridoma cells reached densities of $8 \times 10^5$ to $1 \times 10^6$ cells/ml, the cells were subcultured and planted with 50% of Iscove's DME medium and 50% of the serum-free medium. The cells were removed from the latter medium by centrifugation at $200 \times g$ for five minutes. The Iscove's DME medium was mixed with 50% of the serum-free medium to form a 50:50 mixture, in which the cell pellet was suspended and then counted. An appropriate amount of cell suspension was planted in the vessel with 50% Iscove's DME and 50% serum-free medium. The planted cell densities preferably do not fall below $5 \times 10^4$ cells/ml and not exceed $1 \times 10^5$ cells/ml.

3. After two to three days post-planting, or when the cell density reached $8 \times 10^5$ to $1 \times 10^6$ cells/ml, the cells were refed with 50% Iscove's DME and 50% serum-free medium.

4. Step 3 was repeated for another passage.

5. After two to three days in culture or when the cell density reached $8 \times 10^5$ to $1 \times 10^6$ cells/ml and viability was about 85%, the cells were cultured on serum-free medium only. When the cells were planted in the serum-free medium for the first time the cell densities were between $1 \times 10^5$ to $8\text{-}9 \times 10^5$ cells/ml.

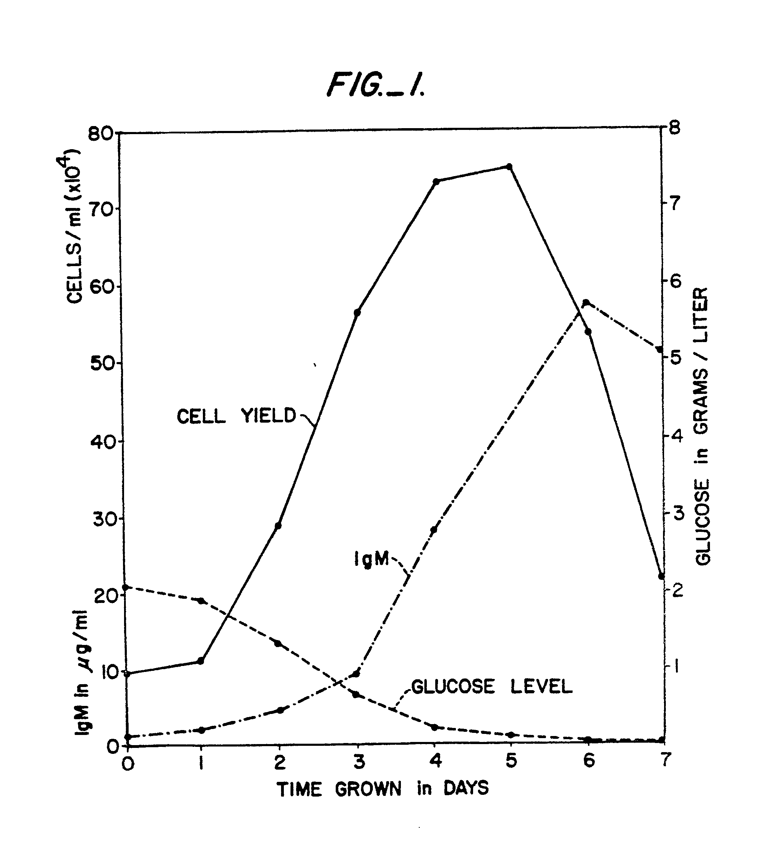

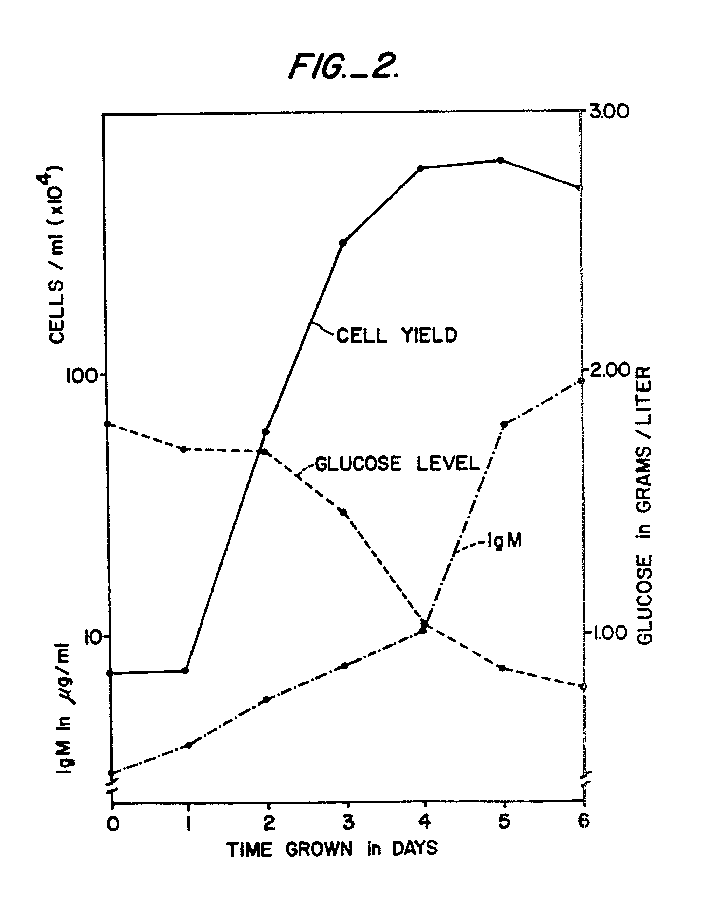

Figure 1 shows the growth curve of D-234 cells in serum-free medium HL-1 (Ventrex). Figure 2 shows the growth curve of D-267 cells in serum-free medium HB104 (Hana Biologicals). Under these conditions, as much as 50-75 $\mu$g/ml of specific human monoclonal antibody was produced.

Previous work by Nelles and Niswander (1984) Infect. Immun. 46:677-681, and Mutharia et al. (1984) Infect. Immum. 45:631-636, demonstrated that mouse monoclonal antibodies reactive with the Rc-J5 mutant of E. coli exhibited extensive cross-reactivity with other gram-negative bacterial species. The studies herein demonstrate the feasibility of generating human monoclonal antibodies that react with cross-reactive determinants of the LPS core region, including those related to the biologically reactive lipid A moiety. One or more of these antibodies may be useful as a novel therapeutic agent in gram-negative sepsis.

Of the sixteen hybrids, D234, D267 and the 'W' series of produced antibodies were considered the best and thus were deposited at the American Type Culture Collection (ATCC), 12301 Parklawn Drive, Rockville, Maryland, USA. Deposit dates and accession numbers are given below:

| Hybridoma | Deposit Date | Accession No. |
|---|---|---|
| 234-4-27-8 | August 10, 1984 | HB 8598 |
| 267-22-49 | August 23, 1984 | HB 8607 |
| WI-3A | February 28, 1985 | HB 8735 |
| WI-4A | February 28, 1985 | HB 8733 |
| WI-5A | February 28, 1985 | HB 8736 |
| WI-6D | February 28, 1985 | HB 8734 |

In addition, the mouse x human fusion partner F3B6 adapted to 99% serum-free medium which partner was the source of these hybridomas was deposited with the ATCC, with the deposit date and accession number given below:

| Fusion Partner | Deposit Date | Accession No. |
|---|---|---|
| F3B6 | April 18, 1985 | HB 8785 |

**Claims**

1. A human antibody which:
   (a) has a substantially homogeneous population;
   (b) binds strongly to determinants that are defined by the lipid A of the cell wall lipopolysaccharides of either E. coli Rc mutants or Salmonella Re mutants;
   (c) binds to either the E. coli Rc mutant lipid A determinants or the Salmonella Re mutant lipid A determinants in intact LPS and in whole gram-negative bacteria;
   (d) blocks the adverse biological effects of gram-negative bacteria endotoxin; and
   (e) exhibits the above properties (a) to (d) at substantially the same extent as the antibody produceable by any of the hybridomas obtainable from ATCC under deposition numbers HB 8598, HB 8607, HB 8735, HB 8733, HB 8736 or HB 8734.

2. An antibody according to Claim 1 which is an IgM.

3. An antibody according to Claim 1 or Claim 2 as obtainable from a hybridoma obtainable from ATCC under deposition number HB 8598, HB 8607, HB 8735, HB 8733, HB 8736 or HB 8734.

4. A stable, permanent hybrid cell line which produces an antibody according to Claim 1 or Claim 2 or progeny thereof.

5. A hybrid cell line selected from those deposited with ATCC under deposition numbers HB 8598, HB 8607, HB 8735, HB 8733, HB 8736 and HB 8734.

6. A pharmaceutical or veterinary formulation for treating bacteremia or sepsis comprising an antibody according to any one of Claims 1 to 3 formulated for therapeutic use and optionally including a pharmaceutically or veterinarily acceptable parenteral vehicle.

7. A formulation as claimed in Claim 6 comprising a plurality of distinct antibodies according to any one of Claims 1 to 3, each of said antibodies binding to a different determinant.

8. A method, other than a method for the therapeutic treatment of a human or an animal, for producing an antibody as defined in Claim 1 comprising growing a cell line according to Claim 4 or Claim 5 in vitro or in vivo to enable said cell line to generate said antibody.

9. A method of producing a cell line defined in Claim 4 comprising forming hybrids using tumor cells and human antibody-generating cells which are capable of generating antibodies against E. coli Rc or Salmonella Re lipid A and selecting and developing hybrid cells which bind to either of said lipid A's and intact LPS and whole gram-negative bacteria.

10. A method of producing a formulation as defined in Claim 6 comprising formulating an antibody according to any one of Claims 1 to 3 for therapeutic use, optionally together with a therapeutically acceptable parenteral vehicle.

**Revendications**

1. Anticorps humain qui :
   (a) a une population sensiblement homogène ;
   (b) se lie fortement aux déterminants qui sont définis par le lipide A des lipopolysaccharides de la paroi cellulaire soit des mutants E. coli Rc, soit des mutants Salmonella Re ;
   (c) se lie soit aux déterminants du lipide A des mutants E. coli Rc, soit aux déterminants du lipide A des mutants Salmonella Re dans les LPS intacts et dans les bactéries Gram négatif entières ;
(d) bloque les effets biologiques indésirables des endotoxines des bactéries Gram négatif ; et
(e) présente les propriétés (a) à (d) ci-dessus sensiblement dans la même mesure que l'anticorps qui peut être produit par l'un quelconque des hybridomes que l'on peut se procurer auprès de l'ATCC sous les numéros de dépôt HB 8598, HB 8607, HB 8735, HB 8733, HB 8736 ou HB 8734.

2. Anticorps selon la revendication 1 qui est une IgM.

3. Anticorps selon la revendication 1 ou la revendication 2 pouvant être obtenu à partir d'un hybridome que l'on peut se procurer auprès de l'ATCC sous les numéros de dépôt HB 8598, HB 8607, HB 8735, HB 8733, HB 8736 ou HB 8734.

4. Lignée cellulaire hybride stable permanente qui produit un anticorps selon la revendication 1 ou la revendication 2, ou une descendance de celle-ci.

5. Lignée de cellules hybrides choisie parmi celles déposées à l'ATCC sous les numéros de dépôt HB 8598, HB 8607, HB 8735, HB 8733, HB 8736 et HB 8734.

6. Formulation pharmaceutique ou vétérinaire pour le traitement de la bactériémie ou de la septicémie, comprenant un anticorps selon l'une quelconque des revendications 1 à 3 formulé pour l'utilisation thérapeutique et acceptable pour l'emploi pharmaceutique ou vétérinaire.

7. Formulation selon la revendicatin 6 comprenant plusieurs anticorps distincts selon l'une quelconque des revendications 1 à 3, chacun desdits anticorps se liant à un déterminant différent.

8. Procédé autre qu'un procédé pour le traitement thérapeutique de l'homme ou d'un animal, pour produire un anticorps tel que défini dans la revendication comprenant la culture d'une lignée cellulaire selon la revendication 4 ou la revendication 5 in vitro ou in vivo pour permettre à ladite lignée cellulaire de produire ledit anticorps.

9. Procédé de production d'une lignée cellulaire définie dans la revendication 4 comprenant la formation d'hybrides, par emploi de cellules tumorales et de cellules humaines productrices d'anticorps qui sont capables de produire des anticorps contre le lipide A d'E. coli Rc ou de Salmonella Re, et la sélection et le développement des cellules hybrides qui se lient soit auxdits lipides A, soit aux LPS intacts, soit aux bactéries Gram négatif entières.

10. Procédé de production d'une formulation telle que définie dans la revendication 6 comprenant la formulation d'un anticorps selon l'une quelconque des revendications 1 à 3 pour l'utilisation thérapeutique avec un véhicule parentéral thérapeutiquement acceptable.

## Patentansprüche

1. Menschlicher Antikörper, der:
   (a) eine im wesentlichen homogene Population aufweist;
   (b) stark an Determinanten bindet, die durch das Lipid A der Zellwand-Lipopolysaccharide von entweder E. coli Rc-Mutanten oder Salmonella Re-Mutanten definiert sind;
   (c) entweder an die E. coli Rc-Mutante-Lipid A-Determinanten oder an die Salmonella Re-Mutante-Lipid A-Determinanten in intaktem LPS und in vollständigen gram-negativen Bakterien bindet;
   (d) die nachteiligen, biologischen Wirkungen von Endotoxin aus gram-negativen Bakterien blockiert; und
   (e) die vorstehenden Eigenschaften (a) bis (d) im wesentlichen im selben Ausmaß aufweist wie der Antikörper der durch eines der von der ATCC unter den Hinterlegungs-Nummern HB 8598, HB 8607, HB 8735, HB 8733, HB 8736 oder HB 8734 erhältlichen Hybridome ist.

2. Antikörper nach Anspruch 1, der ein IgM ist.

3. Antikörper nach Anspruch 1 oder 2, der von einem von der ATCC unter den Hinterlegungsnummern HB 8598, HB 8607, HB 8735, HB 8733, HB 8736 oder HB 8734 erhältlichem Hybridom, erhältlich ist.

4. Stabile, permanente Hybridzellinie, die einen Antikörper nach Anspruch 1 oder 2 erzeugt oder Nachkommen davon.

5. Hybridzellinie, ausgewählt aus den bei der ATCC unter den Hinterlegungsnummern HB 8598, HB 8607, HB 8735, HB 8733, HB 8736 und HB 8734 hinterlegten.

6. Pharmazeutische oder veterinärmedizinische Formulierung zur Behandlung von Bacteriämie oder Sepsis, umfassend einen zur therapeutischen Verwendung formulierten Antikörper nach einem der Ansprüche 1 bis 3 und gegebenenfalls einen pharmazeutisch oder veterinärmedizinisch verträglichen parenteralen Träger.

7. Formulierung nach Anspruch 6, umfassend eine Vielzahl von verschiedenen Antikörpern nach einem der Ansprüche 1 bis 3, wobei jeder der Antikörper an eine unterschiedliche Determinante bindet.

8. Verfahren, das kein Verfahren zur therapeutischen Behandlung eines Menschen oder eines Tieres ist, zur Herstellung eines Antikörpers nach Anspruch 1, umfassend das Züchten einer Zellinie nach Anspruch 4 oder 5 in vitro oder in vivo, um der Zellinie die Erzeugung des Antikörpers zu ermöglichen.

9. Verfahren zur Herstellung einer Zellinie nach Anspruch 4, umfassend die Bildung von Hybriden mit Tumorzellen und menschlichen Antikörper erzeugenden Zellen, die zur Erzeugung von Antikörpern gegen E. coli Rc-oder Salmonella Re-Lipid A befähigt sind, und die Selektion und Entwicklung von Hybridzellen, die entweder an die Lipide A, intaktes LPS oder vollständige gram-negative Bakterien binden.

10. Verfahren zur Herstellung einer Formulierung nach Anspruch 6, umfassend die Formulierung eines Antikörpers nach einem der Ansprüche 1 bis 3 zur therapeutischen Verwendung, gegebenenfalls zusammen mit einem therapeutisch verträglichen, parenteralen Träger.

18

## FIG._1.

# FIG._2.